# EUROPEAN PATENT SPECIFICATION

(11) **EP 4 355 850 B1**
(45) Date of publication and mention of the grant of the patent: **19.03.2025**
(21) Application number: 22751172.2
(22) Date of filing: 15.06.2022
(51) Int. Cl.: C12M 1/00, C12M 1/107

(54) **INTEGRATED PROCESS FOR THE SUSTAINABLE AND AUTONOMOUS CO2-EMISSION-FREE PRODUCTION OF HYDROGEN AND RELATED SYSTEM**
INTEGRIERTES VERFAHREN ZUR NACHHALTIGEN UND AUTONOMEN CO2-EMISSIONSFREIEN HERSTELLUNG VON WASSERSTOFF UND ZUGEHÖRIGES SYSTEM
PROCÉDÉ INTÉGRÉ POUR LA PRODUCTION DURABLE ET AUTONOME SANS ÉMISSION DE CO2 D'HYDROGÈNE ET SYSTÈME ASSOCIÉ

(30) Priority: 16.06.2021 IT 202100015770
(43) Date of publication of application: 24.04.2024
(73) Proprietor: Ventriglia, Fausto Maria, 00046 Grottaferrata (RM) (IT)
(72) Inventor: Ventriglia, Fausto Maria, 00046 Grottaferrata (RM) (IT)
(74) Representative: Santi, Filippo
(86) International application number: PCT/IT2022/050172
(87) International publication number: WO 2022/264182

(56) References cited:
- WO-A1-2020/191442
- US-A1- 2009 321 349
- US-A1- 2013 181 460

## Description

The present invention concerns an integrated process for the sustainable and autonomous production of hydrogen from algal biomass and the related system. In particular, the present invention concerns an integrated renewable process for producing hydrogen wherein four subprocesses are used which, suitably integrated with each other, make it possible to obtain pure hydrogen, the so-called green hydrogen, without CO₂ emissions and by recycling the by-products generated by the various processes.

The fight against climate change is a fundamental political objective of almost all the world's countries, whose primary actions are focused on the drastic reduction of emissions of (direct, indirect, industrial and from combustion) CO₂. To this purpose, the use of hydrogen, especially in combustion processes, is strongly recommended and is considered one of the future sources for mobility: it must not be considered a source of energy but an "energy carrier", i.e. a means for accumulating and transporting energy to the final user.

However, the production of hydrogen is itself a source of high CO₂ emissions. The current technological research focuses on processes for producing the so-called "green" hydrogen, i.e. without CO₂ emissions.

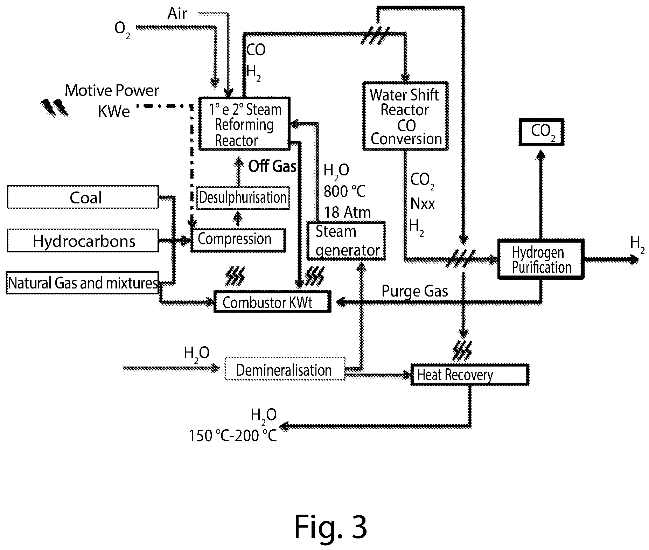

As is well known, roughly 90% of worldwide hydrogen is produced with the chemical process of *Steam Reforming* (shown in Figure 3). It is based on the transformation at high temperature (over 800°C) of coal and/or hydrocarbons in general, and more particularly methane, by reaction with water vapor. It is a known and long-used technology which has been adapted to individual application cases, mainly downstream of oil refining plants. This process has the drawback of producing huge quantities of CO₂, which is usually released into the atmosphere. In this case, the produced hydrogen is defined as "black" or "grey".

More recently, systems for capturing and storing the produced CO₂ have been applied: in this case, the produced hydrogen is defined as "blue". Given the high temperature of the process, usually obtained by burning part of the same hydrocarbon necessary for the chemical transformation, the *Steam Reforming* systems release (and waste) large quantities of thermal energy in the form of heat at 150°C - 200°C in steam mode.

The main primary transformation reaction of a reforming reaction with steam is:

CH₄ + H₂O → CO + 3H₂

The mixture of the primary reactor contains CH₄, H₂O, H₂ and CO. Through a further post-reaction with air, a further transformation of CH₄ is obtained according to the following reactions:

CH₄ + 2O₂ → CO₂ + 2H₂O

2H₂ + O₂ → 2H₂O

CH₄ + H₂O → CO + 3H₂

CH₄ + CO₂ → 2CO + 2H₂

In order to increase the hydrogen yield, the *Steam Reforming* process is followed by a process called *Water Gas Shift Reaction* transforming the produced carbon monoxide CO fractions into CO₂ and H₂ (CO + H₂O → CO₂ + H₂) in the presence of specific catalysts.

As mentioned above, such technology has the drawback of producing huge quantities of CO₂, equal to about 9.5 kg of CO₂ per kg of produced hydrogen, and of dissipating large quantities of thermal energy.

A further process used for producing hydrogen is the water electrolysis process by alkaline hydrolysis, an also known and long time used process. The direct injection of electric current into the water allows its splitting into gaseous fractions of hydrogen and oxygen according to the following elementary reaction

H₂O → H₂O + H⁺ + OH⁻

in free dissociation equilibrium.

By applying an electric field, we obtain:

2H⁺ + 2e⁻ → H₂

40H⁻ - 4e⁻ → 2H₂O + O₂

The industrial use of the alkaline electrolysis process requires the use of suitable salts, such as KOH, promoting electric conductivity, and suitable membranes avoiding direct contact of gases and eliminating the risk of explosions. The more efficient the alkaline electrolysis process, the higher the applied current density; moreover, the process is strongly affected by the temperature and phase of water, allowing higher yields as the higher the temperature and pressure are. In industrial electrolysers, such temperature increase is obtained by using part of the electric power injected.

This process, in addition to being less efficient (economically and energetically) than the chemical process of *Steam Reforming,* depends on the "quality" of the electric power used for electrolysis. The use of electric power from traditional fossil sources or from biomass combustion involves CO₂ emissions into the atmosphere and consequently the produced hydrogen must be defined as "grey"; vice versa, the use of electric power from completely renewable and CO₂ emission-free sources, such as wind, hydroelectric or photovoltaic power produces "green" hydrogen, i.e. without CO₂ emissions. In this case, the subsequent combustion of this type of hydrogen (generating only water as a by-product) does not contribute, even indirectly, to CO₂ emissions into the atmosphere.

However, even this hydrogen production scheme, i.e. "energy from renewable source + electrolysers", indirectly induces CO₂ emissions (so-called secondary) and generates other environmental issues related to the renewable source. In particular:
- in the case of photovoltaics, the production of a module requires about 3,500 kWh/kWp and the emission of about 350 g/kWp of CO₂ (especially in the production step of the "solar grade" silicon ingot with the Siemens process and the Czochralski process) and, therefore, there is the issue of the "renewability" of the primary source of energy used to produce the photovoltaic module itself; other issues related to the use of photovoltaic power are the large amount of land required, the efficiency decay over time and the disposal of the plants at the end of their life;
- in the case of wind power, the energy required for the production and installation of a wind turbine is even higher than in the photovoltaic case (over 5,000 kWh/kWp) and the environmental impact in terms of occupied space is considerable;
- the hydroelectric energy production requires constructing water containment dams for which huge quantities of cement are used whose industrial production process is responsible for the greater CO₂ emissions into the atmosphere, in addition to requiring significant amounts of energy, even if lower than the two previous processes; despite the remarkable efficiency of the hydroelectric basins, however they have a substantial environmental impact.

For each of the three cases illustrated above, it is also important to consider the economic aspect of amortisation of the investments required, which must be added to the, no less important, investment for the electrolysers technology.

There are other hydrogen production processes (by thermochemical, direct biological, nuclear procedures) whose development stage is still experimental or whose characteristic is not the absence of CO₂ emissions. Document US2013181460 describes a process for the production of hydrogen using algal biomass produced in a photobioreactor, combined with reforming of steam, methane and oxygen and alkaline electrolysis of water, The carbon dioxide required for the culture of algae is separated from the gases produced by steam reforming.

In the light of the above, the need to provide new hydrogen production processes that are sustainable and able to overcome the drawbacks of the known processes, in particular with respect to CO₂ emissions and energy consumption, appears evident.

The solution according to the present invention fits in this context, aiming to provide an integrated and circular process for the sustainable and autonomous production of high-efficiency green hydrogen.

In particular, the process according to the present invention allows the production of pure, so-called green, hydrogen without CO₂ emissions into the atmosphere, as the CO₂ is continuously reused in the process.

Furthermore, the process according to the present invention allows to achieve the following advantages compared to the known production processes mentioned above:
- it does not require any external energy source, except that to support operation of service equipments;
- it does not emit primary and secondary CO₂;
- it generates sustainable by-products usable in agriculture (whose industrial production usually involves additional CO₂ emissions);
- in the event that the process includes the use of photovoltaic generators, it allows to double the yield per unit of occupied area;
- it is fundamentally based on an agricultural process and allows sustainable cultural impacts on the territory and a lower environmental impact.

The aim of the present invention is therefore to realise a process allowing to overcome the limits of the solutions according to the known technology and to obtain the technical results previously described.

A further aim of the invention is that said process can be carried out with substantially low costs, both in terms of investment costs and in terms of production and management costs.

Last but not the least aim of the invention is to create a system that is substantially simple, safe and reliable.

These and other results are obtained through the partial use of four different technological processes, already known for other uses, integrating them with each other in terms of exchange of by-products, thermal energy and electric power.

The four processes totally or partially integrated within the procedure according to the present invention are the following open-cycle processes:
i) the autotrophic proliferation process of microalgae with high lipid content fed with CO₂, usually intended for producing biomethane;
ii) the biological process of anaerobic digestion by means of fermentative enzymes intended for producing biomethane;
iii) the chemical process of *Steam Reforming,* intended for splitting methane into hydrogen, described above; and
iv) the electrochemical process of alkaline electrolysis at high current density (EAHCD) described above.

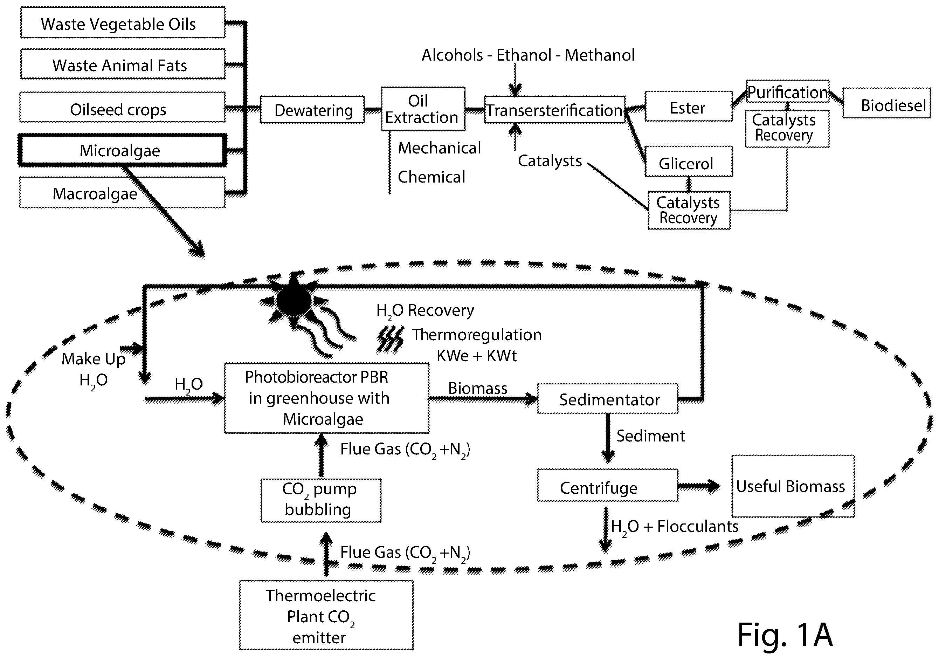

The known process of proliferation of microalgae, shown in Figure 1A, is a sub-process used for producing biodiesel by extracting the lipid mass obtained from the algal biomass. The proliferation of microalgae takes place in a protected and controlled environment, in ponds with a continuous circulation of water. The algae are fed by CO₂ flows introduced and dissolved by bubbling directly into the proliferation water, so that the combination of solar radiation and the photosynthesis process generate further multicellular microalgae (autotrophy). The latter have the characteristic of having a very low residence time and therefore very high proliferation rates, if fed with CO₂ and specific nitrate-based micronutrients. The proliferation ponds, known as photobioreactors, can be either planar protected by greenhouses or not, or they can consist of transparent tubes wherein the proliferation fluid flows. There are many varieties of microalgae (over 50 varieties) suitable for this type of culture, and the technology relating to the culture, growth and harvesting of this type of biomass is now widespread for industrial (pharmaceutical, chemical, etc.), food and energy purposes. Usually, the required CO₂ necessary for proliferation is captured by sources external to the photobioreactor, such as large thermodynamic plants (thermoelectric plants, waste incinerators, etc.). The temperature of vegetation and proliferation of these organisms must be constant and kept around 22°-28 °C according to the type of microalga.

In the biofuel production process, the produced biomass is subjected to a concentration of the lipid part which, with an esterification process, is separated from glycols and then into fuel, as shown in Fig. 1A.

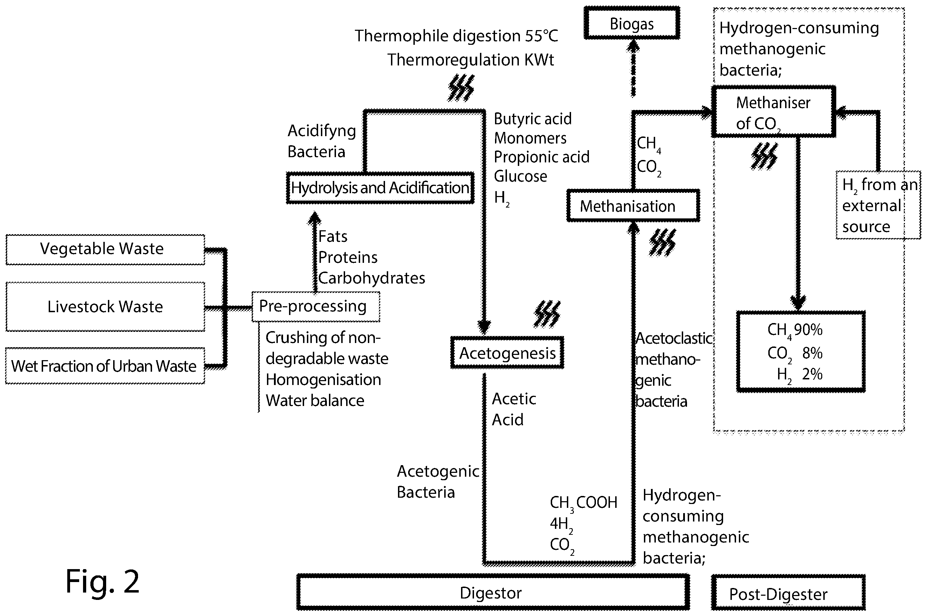

Regarding the known process of anaerobic digestion, shown in Figure 2, this is based on the enzymatic breakdown of biological materials of various kinds (fats, proteins, carbohydrates), mainly of agricultural origin (crop waste, livestock waste, etc.) but also organic waste and wet fractions of urban waste. It consists in the biological reaction of some types of bacteria in an anaerobic environment with said materials, until acetification and consequent methanisation are obtained. enzymatic splitting occurs in 3 phases:
- hydrolysis and acidification: wherein fats, proteins and carbohydrates are split into volatile fatty acids and simple monomers;
- acetification: wherein simple acids (butyric and propionic acid) split into acetic acid;
- methanisation: wherein acetic acid converts into methane CH₄ and carbon dioxide CO₂. In the third phase, two different types of bacteria act, one that acts directly on acetic acid producing CH₄ and CO₂, the other, through a Post-Digester, which recombines the CO₂ into another CH₄ through fractions od hydrogen H₂. The bacteria used can be psychrophilic, mesophilic or thermophilic depending on the growth temperature.

The Anaerobic Digestion process has long been known and mainly used for the valorisation of agricultural residues and waste or even urban waste in order to obtain electric power from Biogas-powered thermodynamic generators, or to power means of transport with Biomethane (if the system provides the latter described).

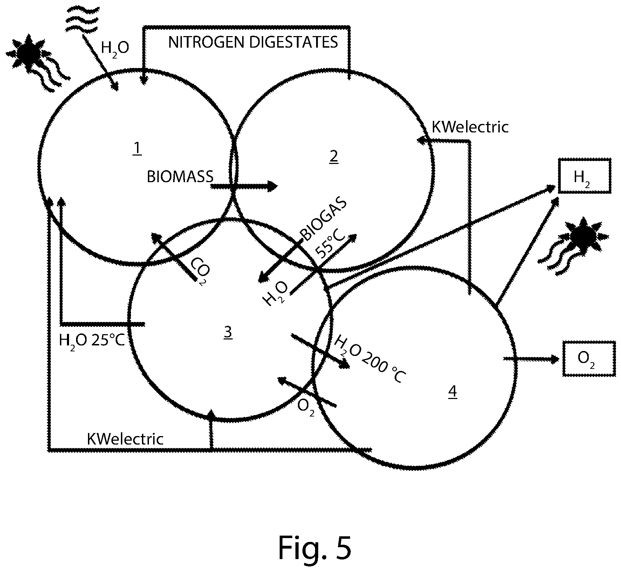

According to the present invention, the four processes mentioned above, or part of them, have been suitably integrated in the exchange of by-products and energy, configuring a series of closed-loop processes, briefly illustrated in Figure 5. The final configuration of the system according to the invention has a surface extension such as to process CO₂ through the proliferation of algal biomass which is transformed biologically and chemically into hydrogen, preferably assisted by a photovoltaic generator. The overall cycle is closed and is advantageously able to reprocess its by-products through the radiant energy of the sun, with a very high final yield.

Therefore, it is a specific object of the present invention an integrated process for producing hydrogen comprising the following steps:
a) production of algal biomass by means of a photobioreactor wherein microalgae are fed with water and carbon dioxide and irradiated with light radiation;
b) anaerobic digestion for obtaining biomethane and nitrogen digestates by means of an anaerobic digester, wherein said anaerobic digestion takes place starting from said algal biomass obtained in said step a);
*c) steam reforming* for obtaining hydrogen, carbon dioxide and heat starting from steam, oxygen and from said biomethane obtained in said step b), and subsequent separation of carbon dioxide; and
d) alkaline electrolysis of water for obtaining hydrogen and oxygen, by means of electrolysers, starting from water heated by the heat obtained in step c) and electric power,
wherein the carbon dioxide in said step a) comes from step c) and the oxygen in said step c) comes from step d).

The water of step a), or microalgae proliferation water, is preferably at a temperature of 25°C and is almost completely recycled (except for losses due to evaporation).

The anaerobic digestion of step b) is preferably an anaerobic digestion of a thermophilic type, more preferably an anaerobic digestion carried out at a temperature of about 55°C. The anaerobic digester is fed with the recovering heat from the *Steam Reforming* step. The residence time of the algal biomass in the anaerobic digester is preferably equal to about 5 times the residence time of the microalgae in the photobioreactor (about 5 days).

The water of step d) comes from external sources. According to the process of the present invention, said nitrogen digestates produced in step b) can be recirculated from said anaerobic digester towards said photobioreactor.

Furthermore, according to the present invention, part of the hydrogen produced in step c) and/or in step d) can be recirculated in said anaerobic digester. In fact, the anaerobic digester can be fed by H₂ fractions taken downstream of the process, i.e. coming from the *Steam Reformer* and the electrolysers, allowing a further transformation of CO₂ into CH₄ and increasing the percentage yield of final biomethane.

Furthermore, according to the present invention, at least part and preferably all of the carbon dioxide produced in step b) and/or in step c) can be recirculated in said photobioreactor.

According to the process of the present invention, the electric power of step d) can come from renewable sources. In particular, the electric power of step d) can be photovoltaic electric power.

A further object of the present invention is an integrated system for implementing a process as defined above, said system comprising
a photobioreactor for producing said algal biomass comprising a carbon dioxide inlet, at least one water inlet and an algal biomass outlet;
an anaerobic digester comprising an algal biomass inlet hydraulically connected to said algal biomass outlet of said photobioreactor, a biomethane outlet, a recovered carbon dioxide outlet and a nitrogen digestate outlet;
*a steam reforming* system, comprising a *reforming* section, followed by a water gas shift section and a section separating hydrogen from carbon dioxide coming from said reforming section (said separation section comprising for example a cryogenic type system, a membrane or PSA system), said *steam reforming* system comprising a biomethane inlet hydraulically connected to said biomethane outlet of said anaerobic digester, a steam inlet and an oxygen inlet, as well as an hydrogen outlet, a carbon dioxide outlet hydraulically connected to the carbon dioxide inlet of said photobioreactor, and one or more heat exchangers designed to heat water inside pipes hydraulically connected to said at least one water inlet of said photobioreactor, to said water inlet of said anaerobic digester and to said water inlet of said electrolysers; and
electrolysers for water electrolysis comprising a water inlet, said water coming from external sources, which is connected to said one or more heat exchangers of said *steam reforming* system, one or more oxygen outlets, at least one of which being hydraulically connected to said oxygen inlet of said *steam reforming* system, and at least one hydrogen outlet.

In a preferred embodiment, said photobioreactor consists of one or more closed tanks. The tanks can be protected by a greenhouse, which prevents water evaporation and keeps temperatures within the algae survival range (thermostating). Furthermore, the tanks can receive the incoming CO₂ through bubblers on the bottom. In particular, in a system according to the invention, to create a photobioreactor for the algal biomass growth, the raceway pond method is preferably used, according to which the photobioreactor consists of a closed tank protected by a greenhouse (Closed Raceway Pond), with ducts for example about 30 cm deep, with a CO₂ recirculating and feeding system by bubbling. Preferably, the tank includes a labyrinth path that allows algae to proliferate along the path.

According to the system of the present invention, said photobioreactor can comprise a nitrogen digestate inlet hydraulically connected to said nitrogen digestate outlet of said anaerobic digester.

Furthermore, according to the system of the present invention, said anaerobic digester can comprise a hydrogen inlet hydraulically connected to said hydrogen outlet of said *steam reforming* system and/or to said at least one hydrogen outlet of said electrolysers.

According to an embodiment of the present invention, said system can comprise a photovoltaic system electrically connected to said electrolysers, to said photobioreactor, to said anaerobic digester and to said steam reforming system by means transmitting electric power.

According to the present invention, said anaerobic digester can comprise a primary digester hydraulically connected to a post-digester.

Furthermore, according to the present invention, said system can further comprise a cooling system, preferably a low enthalpy geothermal cooling system, for thermostating the proliferation waters of the photobioreactor. This allows to avoid the use of an external (or self-produced) motive power.

In particular, the geothermal system consists of a heat exchanger located several meters underground, possibly at groundwater level, where the greenhouse water cools down to the subsoil temperature. It can advantageously contribute to the energy saving of the system according to the invention by cooling the greenhouses in the summer and avoiding to use the energy of the photovoltaic system for this purpose.

The present invention will now be described, for illustrative but not limitative purposes, according to a preferred embodiment thereof, with particular reference to the figures of the attached drawings, in which:
**Figure 1A** shows a diagram of the known microalgae proliferation process;
**Figure 1B** shows a diagram of the algal biomass production step according to the present invention;
**Figure 2** shows a diagram of the known anaerobic digestion process;
**Figure 3** shows a diagram of the known *Steam Reforming* process;
**Figure 4** shows a diagram of an integrated system for realising an integrated process according to the present invention;
**Figure 5** shows a diagram of the integrated process of the present invention as a loop cycle; and

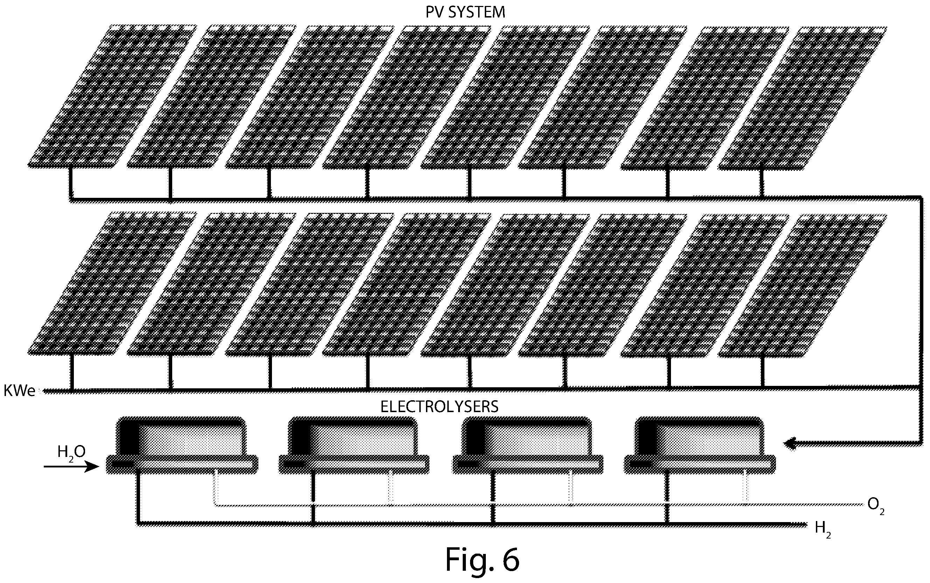

**Figure 6** shows a photovoltaic system electrically connected to electrolysers according to the present invention.

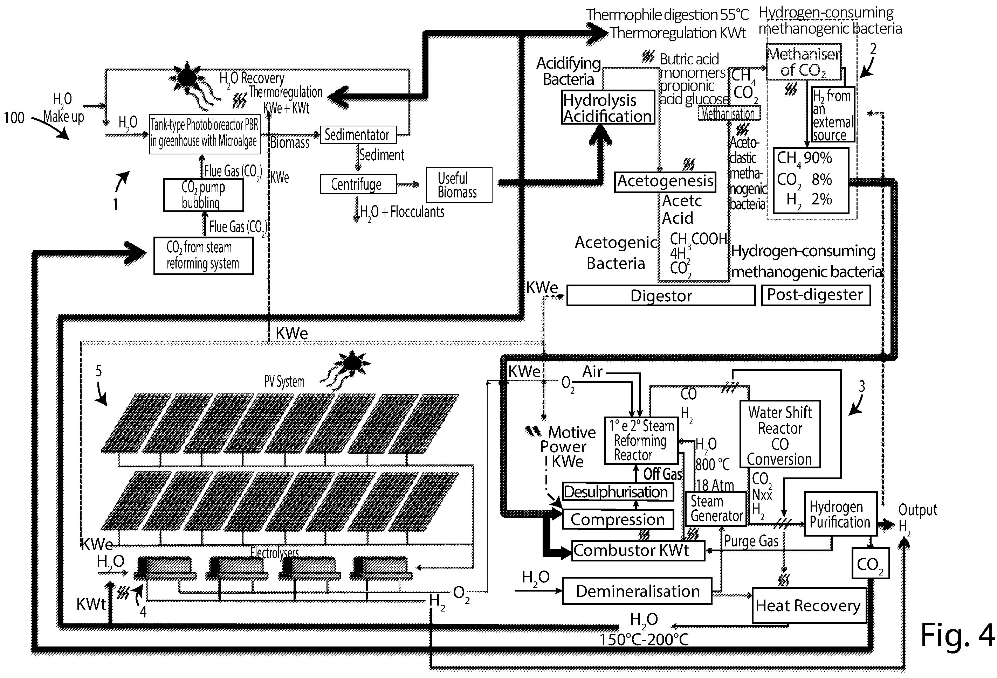

As it can be observed, the combination of the products of the four processes integrated in the system 100 of Figure 4 generates four feedback loops, represented in Figure 5, each fed by the other processes. Together they form a closed loop cycle powered only by solar energy and water, which mainly generates hydrogen, as well as gaseous oxygen and NPK digestate intended for agricultural fertilisation.

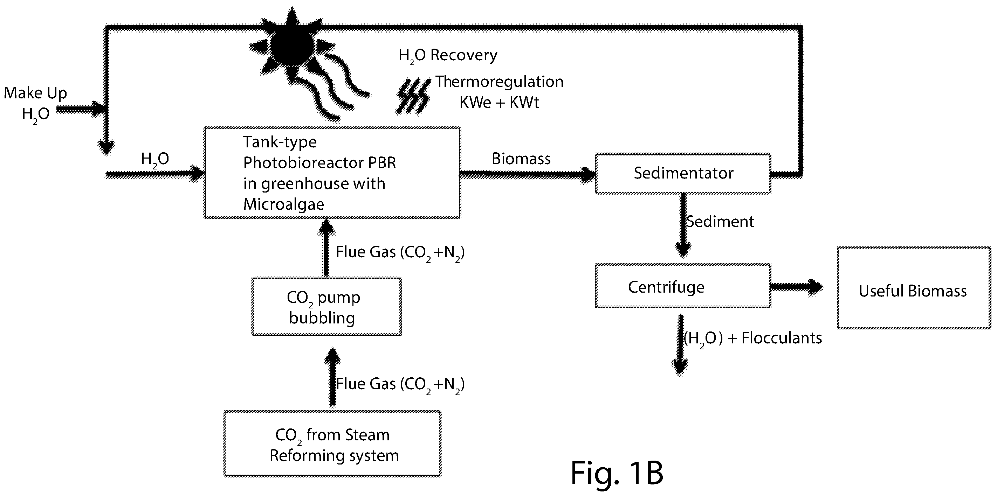

In detail, according to the integrated system 100 shown in Figure 4, the process comprises a step of algal biomass production by means of at least one photobioreactor 1 (i.e. one or more photobioreactors 1), preferably a pond-based photobioreactor 1, or *Raceway Pond,* protected by a greenhouse, wherein the microalgae are fed with water and CO₂ obtaining the biomass production (Fig. 1B). In the process according to the present invention, only the hatched part of the known algae proliferation process shown in Figure 1A, better represented in Figure 1B, is used. In particular, according to the present invention, said photobioreactor 1 (both pond-based and pipe-based, preferably pond-based protected by a greenhouse) is fed with CO₂ by bubbling or with another system for absorbing a gas in water.

As shown in Figure 4, according to the process of the invention, the CO₂ feeding for the microalgae derives from integration with the two subsequent processes, namely the anaerobic digestion step, implemented by means of an anaerobic digester 2 (Figure 2), and the *Steam Reforming* step, implemented by means of a specific *Steam Reforming* 3 or *Steam Reformer* 3 system (Figure 3), which produces CO₂ as a by-product of biomethane splitting coming from said anaerobic digester 2.

In particular, at the end of the proliferation cycle, a certain amount of algal biomass (depending on proliferation factors such as solar radiation, microalga type, water flow rate, etc.) which is wet and, therefore, is preferably subjected to sedimentation and/or centrifugation in order to reduce the quantity of water until reaching the concentration useful for processing the biomass with the subsequent process, i.e. the anaerobic digestion, is extracted from said photobioreactor 1. Therefore, the anaerobic digester 2 is preferably fed by the algal biomass coming from the photobioreactor 1 in the correct and most suitable moisture concentration. The water extracted from sedimentation and/or centrifugation of the algal biomass can be advantageously recovered, possibly filtered, and reused in the photobioreactor 1 (circularity).

Furthermore, as described later, the CO₂ gas feeding said photobioreactor 1 comes from the *Steam Reforming* step, as well as the thermal energy necessary for thermostating the water of the photobioreactors 1. In fact, a part of the waste thermal energy from the *Steam Reforming* system 3, much lower than the total thermal energy produced, can be used for conditioning the recirculation water of the *Raceway Pond 1.* The conditioning of water of the ponds of said photobioreactor 1 can be done through pipe coils deposited in the pond. In particular, heating is required in winter, while the water must be cooled in summer, therefore it is preferable to use geothermal systems with underground heat exchangers.

As shown in Figure 4, the anaerobic digester 2 supplies nitrogen-based (digestate) flours, which can be advantageously used in part to feed the recirculation water of *Raceway Pond* 1 for the microalgae.

As mentioned above, a further step of the integrated process according to the present invention is the anaerobic digestion step. An anaerobic digester 2 according to the system 100 of the present invention is preferably a continuous-type digester (or it can be a batch-type digester) and, as shown in Figure 4, receives the algal biomass, suitably reduced in the correct wet fraction, coming from Photobioreactor 1. Preferably, the anaerobic digester is composed of a primary digester and a post-digester as shown in Figure 2.

Since the anaerobic digestion rate is much faster in case of use of thermophilic bacteria, in particular at a temperature of 55 °-70 °C, any heat requirement can be provided by recycling part of the heat exiting the subsequent *Steam Reforming* process. Furthermore, a negligible fraction of H₂ exiting the *Steam Reforming* process can optionally be an additional supply of H₂ in the anaerobic digestion step, in order to stabilise the CO₂ "methanisation" step.

The final result of the anaerobic digestion is the production of methane gas (or biomethane) in percentages higher than 90% and residual CO₂, which are directed to the subsequent *Steam Reforming* process illustrated in Figure 3. The remaining semisolid bacterial substrate is composed of nitrogen digestate (NPK), which can be used as agricultural fertiliser and soil conditioner (also in hydroponic and aeroponic cultivation methods) or can be recirculated in input towards said photobioreactor 1.

Regarding the *Steam Reforming* step, as shown in Figure 3, the feeding of the *Steam Reformer* 3 takes place with biomethane from the Anaerobic Digester 2 and with water from a source external to the process (for which an appropriate treatment is required). The *Steam Reforming* system 3 uses a small part of the biomethane to produce steam through combustion (whose fumes are appropriately captured and reused), in order to reach the temperatures required by the process, and the remaining part is used for transformation. Through the process already described above, by virtue of the integration with a water gas shift section, the *Steam Reforming* system 3 produces a mixture of hydrogen H₂ gas and carbon dioxide CO₂, which are separated by an appropriate separation system (for example a membrane or PSA system), so that the hydrogen is destined for the final use, while the CO₂ gas (about 9.5 kg per kg of produced hydrogen) is destined to feed the microalgae in the tanks of the photobioreactor 1.

The heat exiting from the *Steam Reforming* 3 step, taken from the low enthalpy steam, is recycled, in particular by means of suitable heat exchangers. In particular, the heat is in part directed to the anaerobic digester 2, in part it is used for heating the microalgae proliferation water (which is preferably at a constant temperature of 25°C) and in large part is used for the electrolyser apparatuses 4 of the alkaline electrolysis step, which use electric power, preferably of photovoltaic origin, to directly produce hydrogen from water.

The integration of the alkaline electrolysis step in the 4-loop cycle according to the process of the invention, allows to feed the electrolysers 4 directly with the heat exiting from the *Steam Reforming* step, which allows to reduce the usual 58 kWh of electric power currently required to produce 1 kg of hydrogen. The electrolysers 4 can be fed with water at increased temperature and pressure, recovering part of the residual heat available from the *Steam Reforming* 3 system, helping to increase the production efficiency of the electrolyser itself. This fourth process finally integrates with the other three described processes it powers (with electric power) and from which it is powered (with thermal energy), helping to produce further hydrogen gas.

The electrolysers 4 also produce pure oxygen which, in addition to being a product commercially intended for the market, can be used to a small extent to stabilise the production of microalgae in the photobioreactor and/or to regulate the operating temperatures of the *Steam Reformer 3.*

According to a preferred embodiment of the process according to the present invention, the electric power necessary to the system is supplied by means of a photovoltaic system 5. In particular, since the photobioreactor 1 consists of a pond protected by a transparent greenhouse having a load-bearing structure, said photovoltaic system 5 can be housed above said photobioreactor 1, in particular on the roof of the aforementioned greenhouse. In particular, a photovoltaic generator of traditional type based on monocrystalline silicon, polycrystalline silicon or thin film, or any other photovoltaic production technology that can be integrated with the greenhouse roof, which preferably does not occupy more than 75% of the greenhouse roof (the photobioreactor 1 itself needs solar radiation to promote the autotrophic algal proliferation), can be housed on said photobioreactor 1.

Said photovoltaic system 5 can advantageously power the electrolysers 4 (of the alkaline type or other equivalent technology) which produce further hydrogen and oxygen gases from further feed water. A part of the electric power produced by said photovoltaic system 5 can be used to power the service equipments of the photobioreactor 1, the anaerobic digester 2 and the *Steam Reforming* system 3, as shown in Figure 4. For example, the photovoltaic system can be used to supply the electric power required to power the paddle agitators and the booster pumps in said photobioreactor 1. Furthermore, the electric power requirement necessary to power the gas separation system can also come from said photovoltaic system 5.

In summary, according to the preferred embodiment of the process and the related integrated system 100 according to the invention shown in the diagram of Figure 4:
- the photobioreactor 1 (or microalgae proliferation greenhouse) supplies biomass to the Anaerobic Digester 2, receiving carbon dioxide and heat from the *Steam Reformer* 3 and receiving nitrogen digestate (fertiliser) from the Anaerobic Digester 2;
- the anaerobic digester 2 supplies methane to the *Steam Reformer* 3, receiving biomass from the Photobioreactor 1, small amounts of hydrogen from the final outlet (therefore from the *Steam Reformer 3* and/or from the electrolysers 4) and heat from the *Steam Reformer 3;*

- the *Steam Reformer* 3 generates pure hydrogen and supplies heat to the electrolysers 4, receiving methane from the Anaerobic Digester 2 and small amounts of oxygen from the electrolysers 4;
- the electrolysers 4 generate pure hydrogen and oxygen by receiving heat from the *Steam Reformer 3;* and
- the photovoltaic system supplies electric power to all other systems.

In a preferred embodiment, the system according to the present invention (or "Hydrogen Farm") is therefore configured as a greenhouse containing growth and proliferation tanks of high lipid content microalgae, on whose roof a photovoltaic system is positioned, and comprising an algae centrifugation system for the biomass concentration, an anaerobic digester for producing biomethane, a *Steam Reforming* system fed by biomethane for producing hydrogen and a CO₂ separation and purification system of cryogenic, membrane or PSA technology type. The system is completed by an electrolysers system powered by photovoltaic power and the heat of the *Steam Reformer.*

Ultimately, the entire four-loop process according to the present invention is based on the production of hydrogen from biomethane and the electrolysis of superheated by-product water using energy from a photovoltaic source. The entire process draws solar energy for the biomass proliferation and for the electric power production in a single integrated system.

The balancing of the various production factors and by-products allows the hydrogen production system according to the present invention to be autonomous and to recycle the by-products, using only negligible quantities of external products (catalysts, flocculants, anticorrosives, etc.).

The invention will be described below for illustrative but not limitative purposes, with particular reference to an illustrative example.

### EXAMPLE 1. Balancing of a four-loop cycle according to the process of the present invention.

Using a variety of stable microalgae such as *Scenedesmus obliquus,* characterised by a medium-high content of lipids (about 40%), we obtain for one m² of photobioreactor (greenhouse) exposed to solar radiation in an area of central Italy at 1,570 kWh/m² per year (photosynthetic efficiency 7%), 14.3 kg/m² per year of biomass (dry weight), requiring 27.9 kg/m² per year of CO₂ (1.987 kgCO₂/kg_{alga}), from which it is obtained about 70% by weight of biogas with a high concentration of methane (90%): 10.0 kg/m² per year of biogas (of which 0.8 kg CO₂), which in *Steam Reforming* generate 2.87 kg/m₂ per year of hydrogen (equal to 31.86 m³/m² per year) and 27.1 kg/m² per year of CO₂ (equal to 97% of the photobioreactor's requirements).

The roof of the greenhouse is 75% covered by commercial photovoltaic panels with an efficiency of around 14.3% (1,350 kWh/kWp per year) with a production of 168.75 kWh/m² per year, 2% of which are intended for direct needs (pumps, lighting, control systems etc.), which the alkaline electrolysers (fed with heat exchanged by superheated steam) transform into 3.48 kg/m² of hydrogen per year (with an efficiency of 20%).

(N.B.: all the counts, for the sake of readability, refer to a standard m² of productive greenhouse)

Therefore, the system returns exactly the CO₂ required by the photobioreactor to feed the microalgae and produces 7.73 kg/m² of hydrogen per year (2.87 + 3.48), equal to 305.0 kWh/m² which compared to irradiation returns an effective overall efficiency of 15.9%, higher than that available from photovoltaic technology.

The *Steam Reforming* system returns a total of 33.0 kWₜₑᵣₘ/m² per year of which 15.96 kWhT/m² per year from convective emissions (165°C/190°C - CO₂, H₂O, CO, H₂, NOₓ) and the rest is radiant energy: the heat is necessary for powering the electrolysers and for the thermostating of the Photobioreactor and the Digester.

## Claims

1. Integrated process for producing hydrogen comprising the following steps:
a) production of algal biomass by means of a photobioreactor (1) wherein microalgae are fed with water and carbon dioxide and irradiated with light radiation;
b) anaerobic digestion for obtaining biomethane and nitrogen digestates by means of an anaerobic digester (2), wherein said anaerobic digestion takes place starting from said algal biomass obtained in said step a);
c) *steam reforming* for obtaining hydrogen, carbon dioxide and heat starting from steam, oxygen and said biomethane obtained in said step b), and subsequent separation of carbon dioxide; and
d) alkaline electrolysis of water for obtaining hydrogen and oxygen, by means of electrolysers (4), starting from water heated by the heat obtained in step c) and electric power,
wherein the carbon dioxide in said step a) comes from step c) and the oxygen in said step c) comes from step d).

2. Process according to claim 1, wherein said nitrogen digestates produced in step b) are recirculated from said anaerobic digester (2) towards said photobioreactor (1).

3. Process according to any one of the preceding claims, wherein part of the hydrogen produced in step c) and/or in step d) is recirculated in said anaerobic digester (2).

4. Process according to any one of the preceding claims, wherein at least part of the carbon dioxide produced in step b) and/or in step c) is recirculated in said photobioreactor (1).

5. Process according to claim 4, wherein all the carbon dioxide produced in step b) and/or in step c) is recirculated in said photobioreactor (1).

6. Process according to any one of the previous claims, wherein the electric power of step d) comes from renewable sources.

7. Process according to claim 6, wherein the electric power of step d) is a photovoltaic electric power.

8. Integrated system (100) for implementing a process as defined in any one of claims 1-7, said system comprising
a photobioreactor (1) for producing said algal biomass comprising a carbon dioxide inlet, at least one water inlet and an algal biomass outlet;
an anaerobic digester (2) comprising an algal biomass inlet hydraulically connected to said algal biomass outlet of said photobioreactor (1), a biomethane outlet, a recovered carbon dioxide outlet and a nitrogen digestate outlet;
a *steam reforming* system (3), comprising a *reforming* section, followed by a water gas shift section and a section separating hydrogen from carbon dioxide coming from said *reforming* section, said steam reforming system (3) comprising a biomethane inlet hydraulically connected to said biomethane outlet of said anaerobic digester (2), a vapor inlet and an oxygen inlet, as well as a hydrogen outlet, a carbon dioxide outlet hydraulically connected to the carbon dioxide inlet of said photobioreactor (1), and one or more heat exchangers designed to heat water inside pipes hydraulically connected to said at least one water inlet of said photobioreactor (1), to said water inlet of said anaerobic digester (2) and to said water inlet of said electrolysers (4); and
electrolysers (4) for water electrolysis comprising a water inlet connected to said one or more heat exchangers of said *steam reforming* system (3), one or more oxygen outlets, at least one of which being hydraulically connected to said oxygen inlet of said *steam reforming* system (3), and at least one hydrogen outlet.

9. System (100) according to claim 8, wherein said photobioreactor (1) comprises a nitrogen digestate inlet hydraulically connected to said nitrogen digestate outlet of said anaerobic digester (2).

10. System (100) according to any one of claims 8-9, wherein said anaerobic digester (2) comprises a hydrogen inlet hydraulically connected to said hydrogen outlet of said *steam reforming* system (3) and/or to said at least one hydrogen output of said electrolysers (4).

11. System (100) according to any one of claims 8-10, wherein said system comprises a photovoltaic system (5) electrically connected to said electrolysers (4), to said photobioreactor (1), to said anaerobic digester (2) and to said steam reforming system (3) by means transmitting electric power.

12. System (100) according to any one of claims 8-11, wherein said anaerobic digester (2) comprises a primary digester hydraulically connected to a post-digester.

13. System (100) according to any one of claims 8-12, said system further comprising a cooling system, for thermostating the proliferation waters of the photobioreactor (1).

## Patentansprüche

1. Integriertes Verfahren zur Herstellung von Wasserstoff, das die folgenden Schritte umfasst:
a) Erzeugung von Algenbiomasse mittels eines Photobioreaktors (1), in dem Mikroalgen mit Wasser und Kohlendioxid gefüttert und mit Lichtstrahlung bestrahlt werden;
b) anaerobe Verdauung zur Gewinnung von Biomethan und Stickstoffverdauungsprodukten mittels eines anaeroben Fermenters (2), wobei die anaerobe Verdauung ausgehend von der in Schritt a) gewonnenen Algenbiomasse erfolgt;
c) *Dampfreformierung* zur Gewinnung von Wasserstoff, Kohlendioxid und Wärme, ausgehend von Dampf, Sauerstoff und dem in Schritt b) gewonnenen Biomethan, und anschließende Abtrennung des Kohlendioxids; und
d) alkalische Elektrolyse von Wasser zur Gewinnung von Wasserstoff und Sauerstoff mit Hilfe von Elektrolyseuren (4), ausgehend von Wasser, das durch die in Schritt c) erhaltene Wärme erhitzt wurde, und elektrischem Strom, wobei das Kohlendioxid im Schritt a) aus dem Schritt c) und der Sauerstoff im Schritt c) aus dem Schritt d) stammt.

2. Verfahren nach Anspruch 1, wobei die in Schritt b) erzeugten Stickstoffabfälle aus dem anaeroben Fermenter (2) in den Photobioreaktor (1) zurückgeführt werden.

3. Verfahren nach einem der vorhergehenden Ansprüche, wobei ein Teil des in Schritt c) und/oder in Schritt d) erzeugten Wasserstoffs in den anaeroben Fermenter (2) zurückgeführt wird.

4. Verfahren nach einem der vorangehenden Ansprüche, bei dem zumindest ein Teil des in Schritt b) und/oder in Schritt c) erzeugten Kohlendioxids in den Photobioreaktor (1) zurückgeführt wird.

5. Verfahren nach Anspruch 4, wobei das gesamte in Schritt b) und/oder in Schritt c) erzeugte Kohlendioxid in den Photobioreaktor (1) rezirkuliert wird.

6. Verfahren nach einem der vorhergehenden Ansprüche, wobei die elektrische Energie von Schritt d) aus erneuerbaren Quellen stammt.

7. Verfahren nach Anspruch 6, wobei die elektrische Energie von Schritt d) eine photovoltaische elektrische Energie ist.

8. Integriertes System (100) zur Durchführung eines Verfahrens nach einem der Ansprüche 1-7, wobei das System umfasst einen Photobioreaktor (1) zur Herstellung der Algenbiomasse, der einen Kohlendioxideinlass, mindestens einen Wassereinlass und einen Auslass für die Algenbiomasse umfasst; einen anaeroben Fermenter (2), der einen Einlass für die Algenbiomasse, der hydraulisch mit dem Auslass für die Algenbiomasse des Photobioreaktors (1) verbunden ist, einen Auslass für Biomethan, einen Auslass für zurückgewonnenes Kohlendioxid und einen Auslass für Stickstoffgärgut umfasst; ein *Dampfreformierungssystem* (3), das einen *Reformierungsabschnitt,* gefolgt von einem Wassergasverschiebungsabschnitt und einem Abschnitt, der Wasserstoff von dem aus dem *Reformierungsabschnitt* kommenden Kohlendioxid trennt, umfasst, wobei das Dampfreformierungssystem (3) einen Biomethaneinlass, der hydraulisch mit dem Biomethanauslass des anaeroben Kochers (2) verbunden ist, einen Dampfeinlass und einen Sauerstoffeinlass sowie einen Wasserstoffauslass, einen Kohlendioxidauslass, der hydraulisch mit dem Kohlendioxideinlass des Photobioreaktors (1) verbunden ist, und einen oder mehrere Wärmetauscher, die dazu bestimmt sind, Wasser in Leitungen zu erhitzen, die hydraulisch mit dem mindestens einen Wassereinlass des Photobioreaktors (1), dem Wassereinlass des anaeroben Kochers (2) und dem Wassereinlass der Elektrolyseure (4) verbunden sind; und Elektrolyseure (4) für die Wasserelektrolyse mit einem Wassereinlass, der mit dem einen oder den mehreren Wärmetauschern des *Dampfreformierungssystems* (3) verbunden ist, einem oder mehreren Sauerstoffauslässen, von denen mindestens einer hydraulisch mit dem Sauerstoffeinlass des Dampfreformierungssystems (3) verbunden ist, und mindestens einem Wasserstoffauslass.

9. System (100) nach Anspruch 8, wobei der Photobioreaktor (1) einen Stickstoff-Gärguteinlass umfasst, der hydraulisch mit dem Stickstoff-Gärgutauslass des anaeroben Kochers (2) verbunden ist.

10. System (100) nach einem der Ansprüche 8-9, wobei der anaerobe Fermenter (2) einen Wasserstoffeinlass umfasst, der hydraulisch mit dem Wasserstoffauslass des *Dampfreformierungssystems* (3) und/oder mit dem mindestens einen Wasserstoffausgang der Elektrolyseure (4) verbunden ist.

11. System (100) nach einem der Ansprüche 8-10, wobei das System ein photovoltaisches System (5) umfasst, das mit den Elektrolyseuren (4), dem Photobioreaktor (1), dem anaeroben Fermenter (2) und dem Dampfreformierungssystem (3) durch Mittel zur Übertragung von elektrischer Energie elektrisch verbunden ist.

12. System (100) nach einem der Ansprüche 8-11, wobei der anaerobe Fermenter (2) einen Primärfermenter umfasst, der hydraulisch mit einem Nachfermenter verbunden ist.

13. System (100) nach einem der Ansprüche 8 - 12, wobei das System ferner ein Kühlsystem zur Thermostatisierung des Proliferationswassers des Photobioreaktors (1) umfasst.

## Revendications

1. Procédé intégré de production d'hydrogène comprenant les étapes suivantes:
a) production de biomasse algale au moyen d'un photobioréacteur (1) dans lequel les microalgues sont alimentées en eau et en dioxyde de carbone et irradiées par un rayonnement lumineux;
b) digestion anaérobie pour obtenir du biométhane et des digestats azotés au moyen d'un digesteur anaérobie (2), dans lequel ladite digestion anaérobie a lieu à partir de la biomasse algale obtenue à l'étape a);
c) *reformage à la vapeur* pour obtenir de l'hydrogène, du dioxyde de carbone et de la chaleur à partir de la vapeur, de l'oxygène et du biométhane obtenu à l'étape b), et la séparation ultérieure du dioxyde de carbone; et
d) électrolyse alcaline de l'eau pour obtenir de l'hydrogène et de l'oxygène, au moyen d'électrolyseurs (4), à partir d'eau chauffée par la chaleur obtenue à l'étape c) et d'énergie électrique, le dioxyde de carbone de l'étape a) provient de l'étape c) et l'oxygène de l'étape c) provient de l'étape d).

2. Procédé selon la revendication 1, dans lequel les digestats azotés produits à l'étape b) sont recirculés dudit digesteur anaérobie (2) vers ledit photobioréacteur (1).

3. Procédé selon l'une quelconque des revendications précédentes, dans lequel une partie de l'hydrogène produit à l'étape c) et/ou à l'étape d) est recirculée dans ledit digesteur anaérobie (2).

4. Procédé selon l'une quelconque des revendications précédentes, dans lequel au moins une partie du dioxyde de carbone produit à l'étape b) et/ou à l'étape c) est recirculée dans ledit photobioréacteur (1).

5. Procédé selon la revendication 4, dans lequel la totalité du dioxyde de carbone produit à l'étape b) et/ou à l'étape c) est recirculée dans ledit photobioréacteur (1).

6. Procédé selon l'une quelconque des revendications précédentes, dans lequel l'énergie électrique de l'étape d) provient de sources renouvelables.

7. Procédé selon la revendication 6, dans lequel l'énergie électrique de l'étape d) est une énergie électrique photovoltaïque.

8. Système intégré (100) pour la mise en œuvre d'un procédé tel que défini dans l'une quelconque des revendications 1 à 7, ledit système comprenant un photobioréacteur (1) pour produire ladite biomasse algale comprenant une entrée de dioxyde de carbone, au moins une entrée d'eau et une sortie de biomasse algale; un digesteur anaérobie (2) comprenant une entrée de biomasse algale connectée hydrauliquement à la sortie de biomasse algale du photobioréacteur (1), une sortie de biométhane, une sortie de dioxyde de carbone récupéré et une sortie de digestat azoté; un système de *reformage à la vapeur* (3), comprenant une section de *reformage,* suivie d'une section de déplacement du gaz de l'eau et d'une section séparant l'hydrogène du dioxyde de carbone provenant de ladite section de *reformage,* ledit système de *reformage à* la vapeur (3) comprenant une entrée de biométhane connectée hydrauliquement à ladite sortie de biométhane dudit digesteur anaérobie (2), une entrée de vapeur et une entrée d'oxygène, ainsi qu'une sortie d'hydrogène, une sortie de dioxyde de carbone récupéré et une sortie de digestat d'azote; et ainsi qu'une sortie d'hydrogène, une sortie de dioxyde de carbone connectée hydrauliquement à l'entrée de dioxyde de carbone dudit photobioréacteur (1), et un ou plusieurs échangeurs de chaleur conçus pour chauffer l'eau à l'intérieur de tuyaux connectés hydrauliquement à ladite au moins une entrée d'eau dudit photobioréacteur (1), à ladite entrée d'eau dudit digesteur anaérobie (2) et à ladite entrée d'eau desdits électrolyseurs (4); et des électrolyseurs (4) pour l'électrolyse de l'eau comprenant une entrée d'eau connectée audit un ou plusieurs échangeurs de chaleur dudit système de *reformage à la vapeur* (3), une ou plusieurs sorties d'oxygène, dont au moins une est connectée hydrauliquement à ladite entrée d'oxygène dudit système de *reformage à la vapeur* (3), et au moins une sortie d'hydrogène.

9. Système (100) selon la revendication 8, dans lequel ledit photobioréacteur (1) comprend une entrée de digestat d'azote connectée hydrauliquement à ladite sortie de digestat d'azote dudit digesteur anaérobie (2).

10. Système (100) selon l'une quelconque des revendications 8 à 9, dans lequel ledit digesteur anaérobie (2) comprend une entrée d'hydrogène connectée hydrauliquement à ladite sortie d'hydrogène dudit système de *reformage à la vapeur* (3) et/ou à ladite au moins une sortie d'hydrogène desdits électrolyseurs (4).

11. Système (100) selon l'une quelconque des revendications 8 à 10, dans lequel ledit système comprend un système photovoltaïque (5) connecté électriquement auxdits électrolyseurs (4), audit photobioréacteur (1), audit digesteur anaérobie (2) et audit système de *reformage à la vapeur* (3) par des moyens transmettant l'énergie électrique.

12. Système (100) selon l'une quelconque des revendications 8 à 11, dans lequel ledit digesteur anaérobie (2) comprend un digesteur primaire relié hydrauliquement à un post-digesteur.

13. Système (100) selon l'une quelconque des revendications 8-12, ledit système comprenant en outre un système de refroidissement, pour thermostater les eaux de prolifération du photobioréacteur (1).
